# EUROPEAN PATENT APPLICATION

(11) **EP 1 992 288 A1**
(43) Date of publication of application: **19.11.2008**
(21) Application number: 07737888.3
(22) Date of filing: 06.03.2007
(51) Int. Cl.: A61B 6/03, A61B 5/055, G06T 1/00, G06T 3/20

(54) **MEDICAL IMAGE PROCESSING METHOD**

(30) Priority: 09.03.2006 JP 2006064624
(71) Applicant: Imagnosis Inc., Kobe-shi, Hyogo 658-0032 (JP)
(72) Inventor: KIM, Han-Joon, Kobe-shi Hyogo 658-0032 (JP)
(74) Representative: Steil, Christian
(86) International application number: PCT/JP2007/054341
(87) International publication number: WO 2007/102509

(57) **Abstract**

It has been desired to provide a method for accurately identifying a medical imaging marker mapped on a display image in correlation with an actual object. According to the present invention, one specific point (e.g., 1b) easy to identify on the display image is selected from a plurality of specific points specified by marker images (1b, 2b, 3b), and correlated with a corresponding marker pattern (1a) present on an actual marker plan diagram (4). Further, the marker images (2b, 3b) specifying the other specific points are successively caused to match in shape and position the corresponding marker patterns (1a, 2a, 3a) on the actual marker plan diagram (4) by translating an image sectional plane (5) perpendicularly to the plane or rotating the image sectional plane (5). Thus, a corrected image marker sectional plane (5') is prepared. This makes it possible to accurately correlate the display image with the actual object.

## Description

### TECHNICAL FIELD

The present invention relates to a processing method for accurately forming a medical image representing a desired part based on medical image data obtained through imaging. Particularly, the present invention relates to a processing method in which a medical imaging marker is utilized and an image of the marker is mapped at a correct position for forming a medical image representing a desired part based on the marker image mapped with its position accurately identified.

### PRIOR ART

In the medical field, medical three-dimensional image information obtained through imaging by CT, MRI or the like is attractive for three-dimensional image diagnosis and simulation of surgical techniques, and positively employed for clinical applications.
However, imaging data is not based on a common coordinate system, and the coordinate system varies among different imaging situations. Further, different imaging apparatuses and imaging means are used for different imaging objects. For example, the CT is adapted for imaging hard tissue such as bones and soft tissue such as skin, while the MRI is mainly adapted for imaging soft tissue. Further, SPECT, PET and the like are mainly adapted for providing an image of a tracer-accumulated part.

In this manner, medical images captured through imaging vary depending on the imaging apparatuses, and are based on different coordinate systems depending on the imaging situations. This makes it difficult to correlate data of one image with data of another image by comparing these images with each other.
That is, it is difficult to provide medical images at the same position in the same orientation based on different sets of data obtained from the same patient at different times or obtained through imaging by different imaging apparatuses (imaging means).

To cope with this, medical imaging markers are used as a common index for correlating the different sets of data with each other. For example, the medical imaging markers are used for correlating different sets of data obtained from the same patient at different times, for example, for evaluating a difference between a pretreatment state and a posttreatment state such as observed before and after a surgery. Further, the medical imaging markers are used for correlating different sets of data obtained by different imaging apparatuses (imaging means), for example, for confirming and evaluating the anatomical position of a tracer-accumulated part imaged by the SPECT or the PET in correlation with a set of data obtained through imaging by the CT or the MRI.

Since the medical imaging markers are used for the aforementioned purposes, the validity of image data for a clinical application is significantly influenced by whether or not marker images (e.g., marker specific points) can be accurately identified.
Further, the accurate identification of the marker images is also important for correlating a captured image with an actual object based on the medical imaging markers for navigation and simulation.

Patent Document 1 discloses a processing method for registering imaged parts present in two different images with each other. In Patent Document 1, markers 1 and 2 respectively present in different images captured on the same imaging sectional plane at different times are detected as reference points, and one of the images is moved to cause the reference points to match each other for registering the images with each other (see paragraphs [0028] to [0030] in Patent Document 1).

Patent Document 2 discloses a method for accurately determining the positional coordinates of a marker within a region of interest (ROI) by manually setting the ROI in the vicinity of the marker.
Patent Document 1: Japanese Unexamined Patent Publication No. 2003-339666
Patent Document 2: Japanese Unexamined Patent Publication No. 2001-170072
Patent Document 3: Japanese Unexamined Patent Publication No. 2006-141640
Patent Document 4: Japanese Patent Application No. 2005-347080

### DISCLOSURE OF THE INVENTION

### PROBLEMS TO BE SOLVED BY THE INVENTION

In Patent Document 1 which discloses the method for registering the imaged parts with each other by registering the marker images with each other for correlating different sets of imaging data obtained at different times, nothing is disclosed or taught about the accurate determination of the positions of the marker images present in the captured images. Particularly, nothing is taught about the accurate determination of the position of the marker image in correlation with the marker attached to the actual object.

In Patent Document 2 which discloses the method for determining the coordinates of the center of the marker by setting the ROI in the vicinity of the marker, the position of the marker in a single captured image is merely determined, and there is no statement about how to use the marker when a plurality of captured images are compared with each other. Particularly, nothing is taught about how to correlate the captured marker image with the marker attached to the actual object.

In view of the foregoing, it is a principal object of the present invention to provide a method for accurately identifying a medical imaging marker mapped on an image in correlation with an actual object.
It is another object of the present invention to provide a method for forming a desired medical image based on a medical imaging marker accurately identified in correlation with an actual object.

### MEANS FOR SOLVING THE PROBLEMS

According to an inventive aspect as set forth in claim 1, there is provided a medical image processing method, which includes the steps of: preparing actual marker information obtained by measuring an actual object provided with a medical imaging marker which specifies at least three points on the object; preparing an actual marker plan diagram based on the actual marker information, the actual marker plan diagram having a plane passing through the three points specified by the marker and including marker patterns which indicate a shape and a position of the marker in the plane; preparing image data obtained by imaging the object provided with the medical imaging marker specifying the at least three points by means of a predetermined imaging apparatus; preparing an image marker sectional plane based on the image data, the image marker sectional plane including marker images which are mapped thereon and each have a shape and a position, the image marker sectional plane passing through three points specified by the mapped marker images; overlaying the prepared actual marker plan diagram on the image marker sectional plane; if not all the marker images mapped on the image marker sectional plane match in shape and/or position the corresponding marker patterns present on the actual marker plan diagram, moving the image marker sectional plane to cause the marker images to match the corresponding marker patterns to prepare a corrected image marker sectional plane which substantially matches the actual marker plan diagram; and specifying, on the corrected image marker sectional plane, spots corresponding to marker patterns present on the overlaid actual marker plan diagram, and identifying the spots as characteristic points of the marker on an image displayed based on the image data.

According to an inventive aspect as set forth in claim 2, the step of preparing the corrected image marker sectional plane includes the step of, if not all the marker images match in shape the corresponding marker patterns on the actual marker plan diagram, translating the image marker sectional plane so that a marker slice position of the image marker sectional plane is shifted perpendicularly to the image marker sectional plane in the medical image processing method as set forth in claim 1.
According to an inventive aspect as set forth in claim 3, the step of preparing the corrected image marker sectional plane includes the step of, if not all the marker images match in shape the corresponding marker patterns on the actual marker plan diagram, rotating the image marker sectional plane so that the marker slice position of the image marker sectional plane is shifted perpendicularly to the image marker sectional plane in the medical image processing method as set forth in claim 1 or 2.

According to an inventive aspect as set forth in claim 4, the step of rotating the image marker sectional plane includes the step of rotating the image marker sectional plane about an axis extending through two points specified by two of the marker images in the medical image processing method as set forth in claim 3.
According to an inventive aspect as set forth in claim 5, the step of rotating the image marker sectional plane includes the step of identifying two of the points specified by the marker images, and moving one of these two points perpendicularly to the image marker sectional plane by inclining a line extending between these two points about the other point with respect to the image marker sectional plane in the medical image processing method as set forth in claim 3.

### EFFECTS OF THE INVENTION

Where a medical image is formed with the use of medical imaging markers, it is a conventional practice to determine the position and the orientation of the medical image based on marker images mapped on the medical image. That is, the slice position and the slice orientation of the medical image are determined based on the marker images mapped on the medical image without strictly evaluating the marker images.
However, the mapped marker images are slightly different in shape and position from the markers depending on the display orientation (angle) and the position of the medical image (particularly in the case of a two-dimensional tomographic image, the depth of an image slice to be displayed).

In the prior art, it is not judged whether the marker images are properly displayed. This makes it impossible to confirm the reliability of the accuracies of specific points specified by the marker images.
In the present invention, the shapes and the positions of the marker images in the image data obtained through the imaging are accurately determined based on the marker patterns indicating the shape and the position of the marker on the actual marker plan diagram prepared from the actual marker information, whereby the accuracies of the positions of the specific points specified by the marker images are improved. As a result, a medical image can be formed as observed at a desired position at a desired angle, so that the medical image can be accurately correlated with the actual object.

In the present invention, the image marker sectional plane is translated and/or rotated for preparing the corrected image marker sectional plane.
In the prior art, all the markers are simultaneously displayed on tomographic images, and the sectional images (tomographic images) are translated to provide an optimum sectional plane (sectional image). In the present invention, on the contrary, one specific point easy to specify on the display image is selected from the plurality of specific points specified by the marker images, and correlated with the corresponding marker specific point present on the actual marker plan diagram. Then, the other specific points are successively caused to match in shape and position the corresponding marker patterns present on the actual marker plan diagram by translating the image marker sectional plane perpendicularly to the plane or rotating the image marker sectional plane, whereby the corrected image marker sectional plane is provided.

Thus, the specific points specified by the marker images based on the image data can be accurately correlated with the specific points specified on the actual marker plan diagram. This makes it possible to correctly identify the positions of the marker images and process the image data based on the correct marker specific points.
After the marker specific points are correctly identified, a reference coordinate system is defined based on the specific points (the at least three specific points). Therefore, an image slice at a desired position, an image slice orientated in a desired direction, a three-dimensional image viewed in a desired direction, and the like can be accurately displayed based on the image data in a reproducible manner.

Further, it is possible to correlate different sets of data obtained from the same patient at different times with each other, to correlate different sets of data obtained by different imaging apparatuses (imaging means), and to correlate the image with the actual object or an entity model. As a result, image diagnosis and image-based surgery navigation and simulation can be accurately performed.

### BRIEF DESCRIPTION OF THE DRAWINGS

Fig. 1 is a diagram illustrating medical imaging markers which are attached to an actual object (patient) to specify at least three points on the object.
Fig. 2 illustrates an exemplary CT display image including marker images 1b, 2b, 3b mapped on a bone image.
Fig. 3A illustrates an exemplary actual marker plan diagram 4 preliminarily prepared.

Fig. 3B illustrates an exemplary display image obtained by overlaying the preliminarily prepared actual marker plan diagram 4 on a prepared image marker sectional plane 5.
Fig. 4 illustrates an exemplary display image of the prepared image marker sectional plane 5.
Fig. 5 illustrates an exemplary display image obtained by overlaying the preliminarily prepared actual marker plan diagram 4 on the displayed image marker sectional plane 5.
Fig. 6 is a diagram for explaining how to operate the image marker sectional plane 5 so as to cause one of three marker patterns 1a, 2a, 3a (e.g., a marker pattern 2a) to match the corresponding marker image 2b.
Fig. 7 is a diagram for explaining how to cause one of the other two marker images (e.g., the marker image 1b) to match the corresponding marker pattern 1a.
Fig. 8 is a diagram for explaining how to rotate (or incline) the image marker sectional plane 5 about an axis extending through a first specific point (the center of the marker image 2b) perpendicularly to the image marker sectional plane 5 so as to change an angle defined between the axis and a straight line L₁₂.
Fig. 9 is a diagram for explaining that the marker images 2b and 1b match the corresponding marker patterns 2a and 1a.
Fig. 10 is a diagram for explaining how to adjust the sectional slice position of the marker image 3b to cause the marker image 3b to match the corresponding marker pattern 3a by rotating the image marker sectional plane 5 about the line L₁₂ extending through the marker images 2b, 1b.
Figs. 11A and 11B are diagrams for explaining a processing operation to be performed as an optional processing operation according to another embodiment of the present invention.
Fig. 12 is a block diagram showing the construction of a computer system 10 to be used for preparing a corrected image marker sectional plane.
Fig. 13 is a flow chart showing steps of a program to be executed by the computer system 10 shown in Fig. 12 for preparing the corrected image marker sectional plane.

### DESCRIPTION OF REFERENCE CHARACTERS

1, 2, 3: Medical imaging markers
1a, 2a ,3a: Marker patterns
1b, 2b, 3b: Marker images
1c, 2c, 3c: Centers of markers 1, 2, 3
4: Actual marker plan diagram
5: Image marker sectional plane
5': Corrected image marker sectional plane

### BEST MODE FOR CARRYING OUT THE INVENTION

With reference to the attached drawings, embodiments of the present invention will hereinafter be described more specifically.

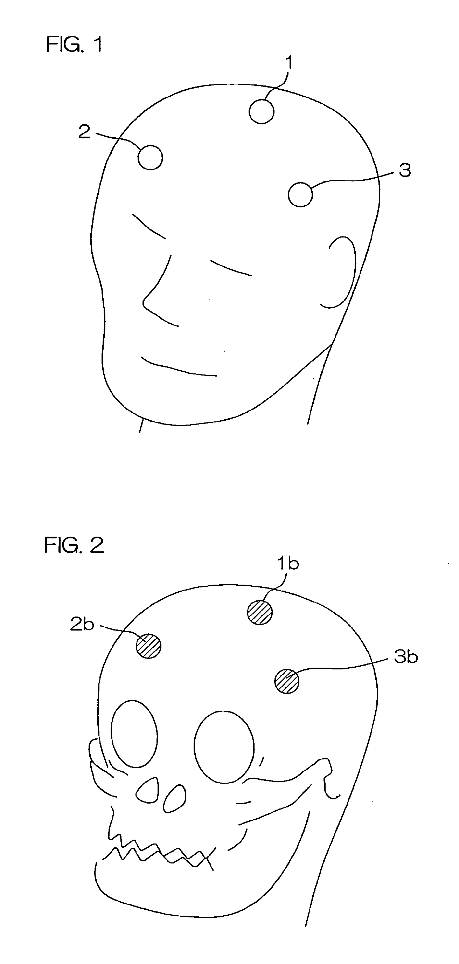

Fig. 1 is a diagram illustrating medical imaging markers (hereinafter each referred to simply as "marker") which are attached to an actual object (patient) to specify at least three points on the object. In this figure, three markers 1, 2, 3 are illustrated as being used for specifying the three points on the object. However, it is not always necessary to use the three markers for specifying the three points, but a marker including three contrasting balls may be used as disclosed in Patent Document 3. In this case, the three points can be specified on the object by the single marker.

The object (patient) provided with the markers for specifying the three points as shown in Fig. 1 is measured, for example, by a three-dimensional measurement apparatus to provide actual marker information including shape/position data of the markers 1, 2, 3 on the object.
While the actual marker information is provided, the object (patient) provided with the markers is imaged, for example, by a CT imaging apparatus. The CT imaging provides a display image as shown in Fig. 2 based on medical image data.

The display image of Fig. 2 is a CT display image including marker images 1b, 2b, 3b mapped on a bone image.
On the other hand, an actual marker plan diagram having a plane passing through the three points specified by the markers 1, 2, 3 and including marker patterns 1a, 2a, 3a which indicate the shapes and the positions of the markers in the plane is prepared based on the actual marker information obtained by measuring the object provided with the three markers 1, 2, 3 as shown in Fig. 1.

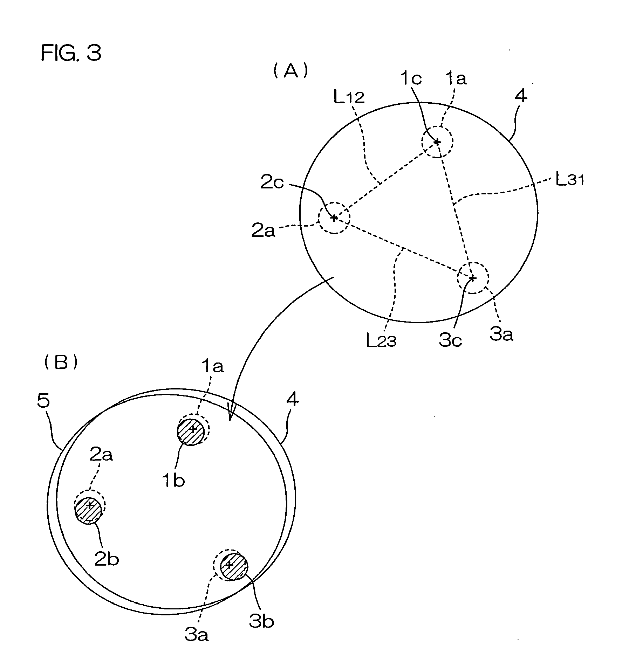

Fig. 3A illustrates an example of the actual marker plan diagram 4 thus prepared. The actual marker plan diagram 4 includes the marker patterns 1a, 2a, 3a which indicate the shapes and the positions of the markers, and lines L₁₂, L₂₃, L₃₁ which connect centers 1c, 2c, 3c of the marker patterns 1a, 2a, 3a to each other. In the actual marker plan diagram 4, the centers 1c, 2c, 3c of the three marker patterns 1a, 2a, 3a are defined as the specific points. Further, the actual marker plan diagram 4 is defined by the plane passing through the three specific points 1c, 2c, 3c.

The actual marker plan diagram 4 shown in Fig. 3A is prepared by processing the shape/position data of the three markers 1, 2, 3 on the object measured, for example, by the three-dimensional measurement apparatus and providing the plane passing through the centers of the three markers 1, 2, 3. The actual marker plan diagram 4 thus prepared may be displayed on a display device by applying data of the actual marker plan diagram to a personal computer.

Subsequently, an image marker sectional plane 5 passing through the three mapped marker images 1b, 2b, 3b and indicating the shapes and the positions of the marker images 1b, 2b, 3b within the plane is prepared from the display image shown in Fig. 2. The image marker sectional plane 5 thus prepared and the preliminarily prepared actual marker plan diagram 4 (Fig. 3A) are displayed in overlapping relation.
An example of the resulting display image is shown in Fig. 3B. The marker patterns 1a, 2a, 3a or the actual marker plan diagram 4 obtained based on the actual shapes and sizes of the markers 1, 2, 3 and the actual positional relationship between the markers 1, 2, 3 are superposed on the mapped marker images 1b, 2b, 3b, whereby the accuracies of the marker specific points can be checked.

When the actual marker plan diagram 4 is superposed on the image marker sectional plane 5, the mapped marker images 1b, 2b, 3b do not necessarily perfectly match in shape and position the corresponding marker patterns 1a, 2a, 3a obtained by the measurement. That is, as shown in Fig. 3B, the marker images 1b, 2b, 3b are generally slightly different in shape from the corresponding marker patterns 1a, 2a, 3a, and slightly offset from the corresponding marker patterns 1a, 2a, 3a.

Therefore, the image marker sectional plane 5 is translated and/or rotated in the following manner so that the marker images 1b, 2b, 3b mapped on the image marker sectional plane 5 matches in shape and position the corresponding marker patterns 1a, 2a, 3a present on the actual marker plan diagram 4.
Where the actual marker plan diagram 4 and the image marker sectional plane 5 are different in magnification ratio from each other, the magnification ratio is corrected based on the magnification ratio of the image obtained by means of the imaging apparatus prior to the matching.

This embodiment is not arranged such that a three-dimensional image formed based on the actual marker information obtained by means of the three-dimensional measurement apparatus and a three-dimensional image obtained, for example, through the CT imaging are compared with each other and superposed one on the other for judging whether or not the marker images mapped through the imaging are different in shape and position from the corresponding actual markers, but arranged such that the two images to be compared are each provided in the form of a plan image (sectional image) for two-dimensional comparison of the shapes and the positions of the marker images and the actual markers.

This is because, where the three-dimensional image is formed based on the obtained image data, it is often impossible to map the marker images in a sufficiently observable form in the three-dimensional image based on the contrast levels of the marker images with respect to the surroundings. In this embodiment, therefore, the positions of the marker images are corrected with reference to the positional relationship between the marker images and the actual marker patterns on the two-dimensional image, i.e., on the plan image (sectional image), which is clearer than the three-dimensional display image.

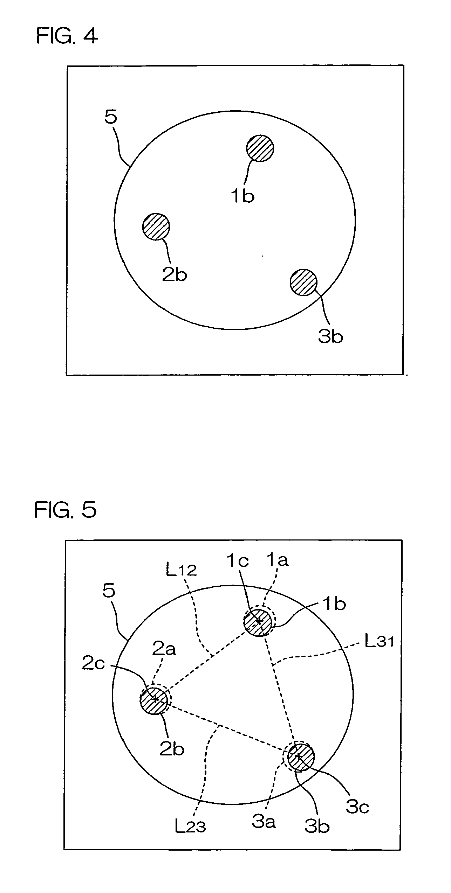

Where the three-dimensional images can be displayed with higher fidelity, the comparison of the shapes and the positions of the marker images and the actual marker patterns is achieved by superposing an actual marker three-dimensional image on an image marker three-dimensional image rather than by preparing the actual marker plan diagram and the image marker sectional plane and superposing the actual marker plan diagram on the image marker sectional plane.
For comparison between the marker images and the actual marker patterns, the prepared image marker sectional plane 5 is first displayed as shown in Fig. 4. The image marker sectional plane 5 includes the marker images 1b, 2b, 3b mapped thereon.

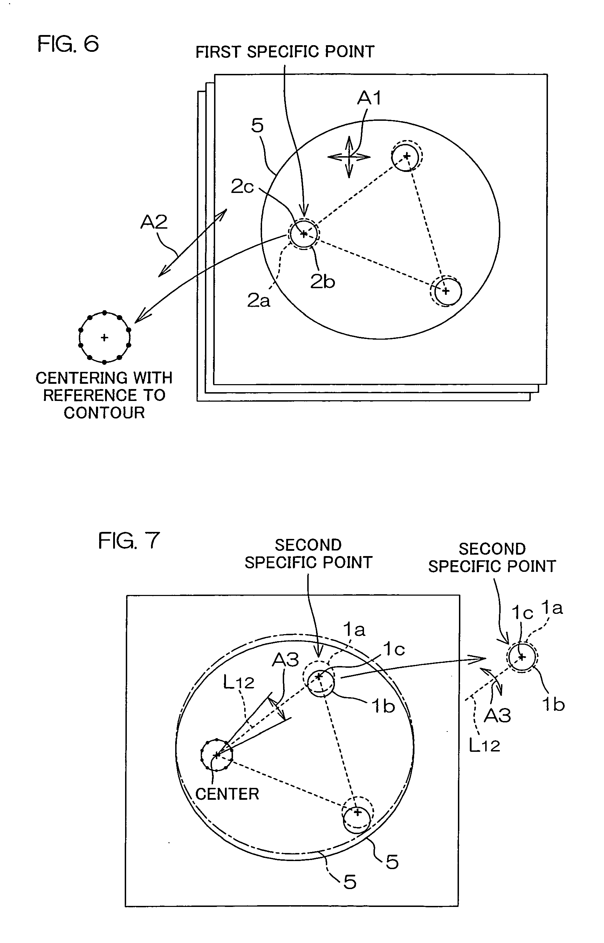

Then, as shown in Fig. 5, the preliminarily prepared actual marker plan diagram 4 is superposed on the displayed image marker sectional plane 5. As a result, the marker images 1b, 2b, 3b do not necessarily match the corresponding marker patterns 1a, 2a, 3a. Therefore, it is common that the marker images 1b, 2b, 3b are different in contour and size from the marker patterns 1a, 2a, 3a, and slightly offset from the marker patterns 1a, 2a, 3a.
Therefore, as shown in Fig. 6, the image marker sectional plane 5 is operated so that one of the three marker patterns 1a, 2a, 3a, e.g., the marker pattern 2a, matches the corresponding marker image 2b. That is, the image marker sectional plane 5 is translated in one of arrow directions A1 within the plane so as to cause the center of the marker image 2b to match the center of the marker pattern 2a. Where the marker image 2b does not match in size the marker pattern 2a, the image marker sectional plane 5 is not located at a proper sectional slice position. Therefore, the image marker sectional plane 5 is translated perpendicularly to the plane for adjusting the sectional slice position of the image marker sectional plane 5. In other words, the sectional slice position of the image marker sectional plane 5 is shifted by translating the image marker sectional plane 5 as indicated by an arrow A2 so that the marker image 2b matches in size the corresponding marker pattern 2a.

With the marker image 2b matching the marker pattern 2a, the specific point (first specific point) specified by the marker 2 is accurately and precisely identified in the image data.
Then, as shown in Fig. 7, a matching operation is performed to cause one of the other two marker images, e. g. , the marker image 1b, to match the corresponding marker pattern 1a.

In this operation, the image marker sectional plane 5 is rotated in one of arrow directions A3 about an axis extending through the first specific point (now defined by the center of the marker image 2b) perpendicularly to the image marker sectional plane 5 to make adjustment such that the line L₁₂ extending through the centers of the marker patterns 2a, 1a passes through the center of the marker image 1b.
Even after this adjustment, there is a possibility that the marker image 1b does not match in size the corresponding marker pattern 1a, or is offset from the corresponding marker pattern 1a on the line L₁₂.

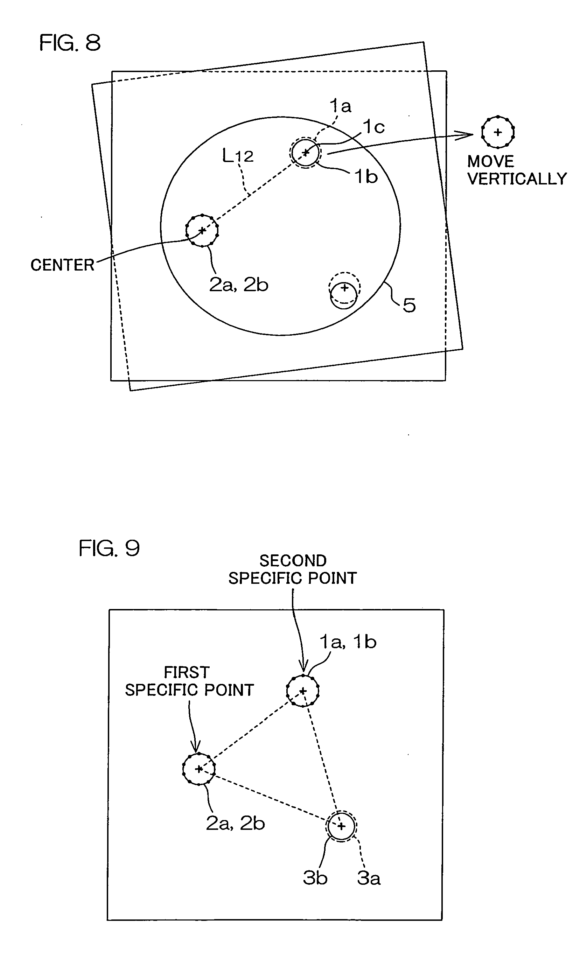

Therefore, as shown in Fig. 8, the image marker sectional plane 5 is moved (inclined) by changing an angle defined between the line L₁₂ and the axis extending through the first specific point (now defined by the center of the marker image 2b) perpendicularly to the image marker sectional plane 5 to move up or down a 1c-side of the line L₁₂ about an intersection between the axis and the line L₁₂ with respect to the paper surface of Fig. 8. Thus, the sectional slice position of the marker image 1b is shifted without any change in the sectional slice position of the marker image 2b to change the size and the position of the marker image 1b, whereby the marker image 1b matches the corresponding marker pattern 1a.

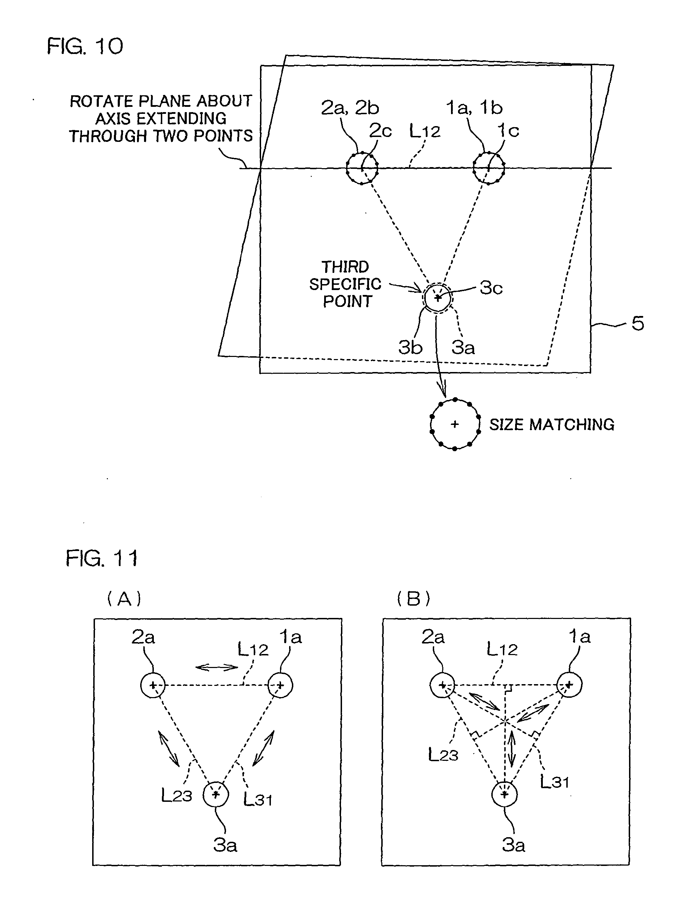

As a result, as shown in Fig. 9, the marker images 2b, 1b match the marker patterns 2a, 1a, respectively.
In this case, the remaining marker image 3b is often different in size from the corresponding marker pattern 3a as shown in Fig. 9.
Therefore, as shown in Fig. 10, the image marker sectional plane 5 is rotated about the line L₁₂ (which now extends through the marker images 2b, 1b) for adjusting the sectional slice position of the marker image 3b to cause the marker image 3b to match the corresponding marker pattern 3a.

As a result, the image marker sectional plane 5 is thus accurately positioned as passing through the three specific points specified by the centers of the three markers 1, 2, 3, and the resulting corrected image marker sectional plane is displayed. Based on the corrected image marker sectional plane 5', a coordinate system for the image data is properly defined, whereby the position and the orientation of the image are accurately reproduced. In addition, the image can be displayed in correct positional and angular relation to the actual object.

The method according to the embodiment described above is such that one marker image selected from the three marker images 1b, 2b, 3b specifying the three points is caused to match the corresponding one of the marker patterns obtained by the measurement, and then the other two marker images are successively caused to match the corresponding marker patterns obtained by the measurement, whereby the three specific points specified by the marker images are accurately identified.
Instead of this method, the image marker sectional plane 5 may be moved along the plane or rotated so as to translate any one of the marker images along a corresponding one of the lines L₁₂, L₂₃, L₃₁ connecting the three marker patterns 1a, 2a, 3a to each other as shown in Fig. 11A.

Alternatively, as shown in Fig. 11B, the image marker sectional plane 5 is moved along the plane or rotated so as to move any one of the marker images along a line extending through the center of the corresponding one of the marker patterns 1a, 2a, 3a perpendicularly to the corresponding one of the lines L₁₂, L₂₃, L₃₁ connecting the three marker patterns 1a, 2a, 3a to each other so that the marker images are adjusted to match the corresponding marker patterns obtained by the measurement.

Further, the image marker sectional plane 5 may be rotated about the gravity center, or the incenter or the circumcenter of a triangle formed by connecting the three marker patterns 1a, 2a, 3a to each other so as to cause the marker images to match the corresponding marker patterns obtained by the measurement.
In any case, the marker images mapped on the medical image data obtained through the imaging are compared with the marker patterns displayed based on the measurement of the positions of the markers on the actual object, and caused to match in size and position with the marker patterns based on the measurement. Thus, the marker images based on the image data are accurately positioned in correlation with the actual markers, and the data of the sectional image to be displayed based on the marker images is uniquely determined and accurately displayed with excellent reproducibility without the fear that its sectional slice position may vary depending on the image data.

That is, the specific points are accurately identified based on the markers, thereby providing a highly reliable medical image. Further, the medical image is highly accurately correlated with the actual object.
The corrected image marker sectional plane 5' described above can be generally automatically prepared by means of a computer system.
In the embodiment described above, spherical markers are used as the markers 1, 2, 3 to be attached to the actual object (human body or patient) by way of example. Optionally, a medical imaging marker disclosed in Patent Document 4 (Japanese Patent Application No. 2005-347080) may be used as any one of the markers 1, 2, 3.

More specifically, the medical imaging marker to be optionally used includes a plate member of a contrasting material which has two flat major surface portions located symmetrically about an intersection of two perpendicular straight lines and each having edges defined by the lines, two pairs of side surfaces provided perpendicularly to the respective maj or surface portions, and boundary edges defined by at least parts of the lines.
With the use of such a medical imaging marker, the resulting marker image per se has directionality, so that the positional relationship of the markers on the object can be more accurately correlated with the positional relationship of the marker images on the captured image by properly displaying the marker images.

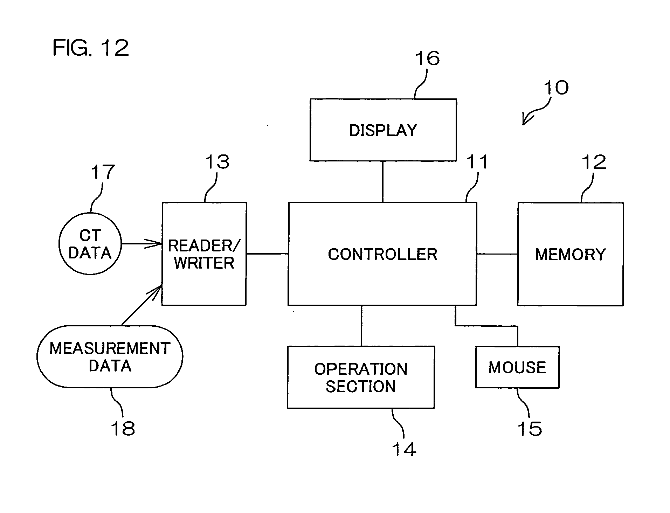

Fig. 12 is a block diagram showing the construction of a computer system 10 to be used for preparing the corrected image marker sectional diagram. Examples of the computer system 10 include personal computer systems and office computer systems which are known in the art.
The system 10 includes a controller 11 including a CPU. The controller 11 is connected to a memory 12 (e.g., a hard disk memory, a solid memory or any other type of memory) , a reader/writer 13, an operating section 14 (e.g., a keyboard or an operation panel), a mouse 15 as an operation member, and a display device 16 (e.g., a liquid crystal display device, a CRT display device or a plasma display device).

When a disk-type storage medium 17, 18, for example, storing CT data or measurement data obtained through measurement by a three-dimensional measurement apparatus is set in the reader/writer 13, the reader/writer 13 reads the CT data or the three-dimensional data from the disk 17, 18, and applies the data to the controller 11.
Further, a program for preparing the corrected image marker sectional plane may be installed in the computer system 10 by utilizing the reader/writer 13.

The computer system 10 installed with the program generally automatically performs the following operation for preparing the corrected image marker sectional plane.

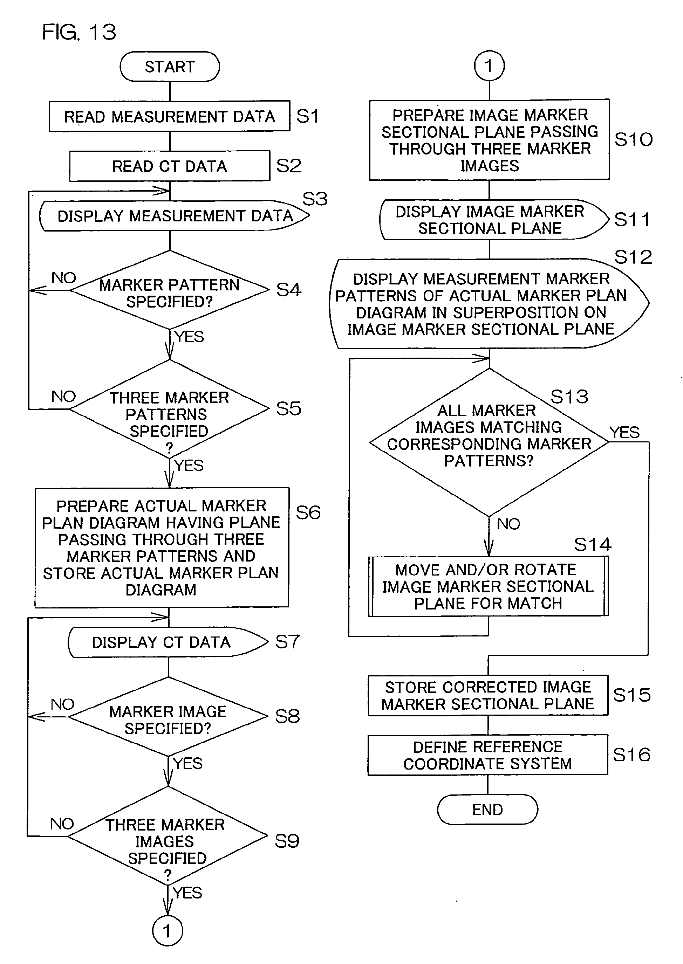

Fig. 13 is a flow chart showing steps of the program to be executed by the computer system shown in Fig. 12 for preparing the corrected image marker sectional plane.
Upon the start of control, three-dimensional measurement data 18 and CT data 17 obtained through imaging are read via the reader/writer 13 (Steps S1 and S2).

The measurement data 18 and the CT data 17 are those obtained by measuring or imaging an actual object provided with markers 1, 2, 3 which specify three points as described with reference to Fig. 1.
Then, the controller 11 processes the measurement data, and displays a measurement data image on the display device 16 based on the processed measurement data (Step S3). As the measurement data image displayed on the display device 16 includes marker patterns 1a, 2a, 3a, a user specifies the marker patterns 1a, 2a, 3a with the use of the mouse 15 (Steps S4, S5). The controller 11 prepares an actual marker plan diagram 4 passing through the specified marker patterns 1a, 2a, 3a (see Fig. 3A), and stores the actual marker plan diagram 4 (Step S6).

In turn, the controller 11 displays a CT data image on the display device 16 based on the CT data (Step S7).
Three marker images 1b, 2b, 3b are mapped on the CT data image displayed on the display device 16 as shown in Fig. 2.
When the user specifies these three mapped marker images 1b, 2b, 3b (Steps S8, S9), the image marker sectional plane 5 passing through the three marker images 1b, 2b, 3b is automatically prepared (Step S10).

Then, the image marker sectional plane 5 thus prepared is displayed on the display device 16 (Step S11), and the marker patterns on the actual marker plan diagram 4 prepared and stored in Step S6 are displayed in superposition on the image marker sectional plane 5 (Step S12).
The resulting display image is shown in Fig. 3B by way of example.
Subsequently, the controller 11 judges whether all the marker images 1b, 2b, 3b match the corresponding marker patterns 1a, 2a, 3a (Step S13). If not all the marker images match the corresponding marker patterns, the image marker sectional plane 5 is moved or rotated so as to cause all the marker images 1b, 2b, 3b to match the corresponding marker patterns 1a, 2a, 3a (Step S14), and the corrected image marker sectional plane 5' is prepared (Step S15). Then, the sectional plane 5' is stored (Step S15).

An operation to be performed in Step S14 is the operation described with reference to Figs. 4 to 10, and automatically performed based on the program.
Alternatively, the user may perform the operation while observing the superposition image displayed on the display device 16. In response to this operation, the controller 11 may prepare the corrected image marker sectional plane 5'.

A reference coordinate system for the CT image data is defined based on the corrected image marker sectional plane 5' thus prepared (Step S16).
After the definition of the reference coordinate system, the CT data image can be displayed in a uniquely determined orientation based on the reference coordinate system with excellent reproducibility.
It should be understood that the present invention be not limited to the embodiments described above, but various modifications may be made within the scope of the present invention defined by the appended claims.

## Claims

1. A medical image processing method comprising the steps of:
preparing actual marker information obtained by measuring an actual object provided with a medical imaging marker which specifies at least three points on the object;
preparing an actual marker plan diagram based on the actual marker information, the actual marker plan diagram having a plane passing through the three points specified by the marker and including marker patterns which indicate a shape and a position of the marker in the plane;
preparing image data obtained by imaging the object provided with the medical imaging marker specifying the at least three points by a predetermined imaging apparatus;
preparing an image marker sectional plane based on the image data, the image marker sectional plane including marker images which are mapped thereon and each have a shape and a position, the image marker sectional plane passing through three points specified by the mapped marker images;
overlaying the prepared actual marker plan diagram on the image marker sectional plane;
if not all the marker images mapped on the image marker sectional plane match in shape and/or position the corresponding marker patterns present on the actual marker plan diagram, moving the image marker sectional plane to cause the marker images to match the corresponding marker patterns to prepare a corrected image marker sectional plane which substantially matches the actual marker plan diagram; and
specifying, on the corrected image marker sectional plane, spots corresponding to marker patterns present on the overlaid actual marker plan diagram, and identifying the spots as characteristic points of the marker on an image displayed based on the image data.

2. A medical image processing method as set forth in claim 1, wherein the step of preparing the corrected image marker sectional plane includes the step of, if not all the marker images match in shape the corresponding marker patterns on the actual marker plan diagram, translating the image marker sectional plane so that a marker slice position of the image marker sectional plane is shifted perpendicularly to the image marker sectional plane.

3. A medical image processing method as set forth in claim 1 or 2, wherein the step of preparing the corrected image marker sectional plane includes the step of, if not all the marker images match in shape the corresponding marker patterns on the actual marker plan diagram, rotating the image marker sectional plane so that the marker slice position of the image marker sectional plane is shifted perpendicularly to the image marker sectional plane.

4. A medical image processing method as set forth in claim 3, wherein the step of rotating the image marker sectional plane includes the step of rotating the image marker sectional plane about an axis extending through two points specified by two of the marker images.

5. A medical image processing method as set forth in claim 3, wherein the step of rotating the image marker sectional plane includes the step of identifying two of the points specified by the marker images, and moving one of these two points perpendicularly to the image marker sectional plane by inclining a line extending between these two points about the other point with respect to the image marker sectional plane.
